# EUROPÄISCHE PATENTSCHRIFT

(11) **EP 1 411 872 B1**
(45) Veröffentlichungstag und Bekanntmachung des Hinweises auf die Patenterteilung: **08.12.2004**
(21) Anmeldenummer: 02764810.4
(22) Anmeldetag: 30.07.2002
(51) Int. Cl.: A61F 2/50, A61F 2/80, A61F 2/60

(54) **PROTHESE**
PROSTHESIS
PROTHESE

(30) Priorität: 02.08.2001 DE 20112884 U; 20.11.2001 DE 20118926 U; 03.05.2002 DE 20207046 U
(43) Veröffentlichungstag der Anmeldung: 28.04.2004
(73) Patentinhaber: Luisa Cerano GmbH, 72622 Nürtingen (DE)
(72) Erfinder: CARSTENS, Johannes, 73730 Esslingen (DE)
(74) Vertreter: Wörz, Volker
(86) Internationale Anmeldenummer: PCT/EP2002/008457
(87) Internationale Veröffentlichungsnummer: WO 2003/013400

(56) Entgegenhaltungen:
- DE-A- 2 718 395
- US-A- 4 872 879
- US-A- 5 728 170
- US-A- 5 888 234
- US-A- 5 931 872
- US-A- 6 106 559
- PATENT ABSTRACTS OF JAPAN vol. 1996, no. 08, 30. August 1996 (1996-08-30) & JP 08 089519 A (KEIAI GISHI ZAIRYO HANBAISHIYO:KK), 9. April 1996 (1996-04-09)

## Beschreibung

Die Erfindung betrifft Prothesen gemäß dem Oberbegriff des Anspruchs 1, im wesentlichen umfassend einen Silikonliner mit Kupplungsstift, einen Prothesenschaft, passend zu einem Gliederstumpf, bereichsweise mit Längsschlitzen versehen und mittels Spannelementen im Durchmesser veränderbar, und einen Halter zum Verbinden einer künstlichen Gliedmaße mit dem Prothesenschaft.

### Stand der Technik

Prothesen haben die Aufgabe, durch Unfall oder Krankheit verlorene Gliedmaßen so weit wie möglich zu ersetzen. Zur zeitweiligen Befestigung der Prothesen, beispielsweise künstlichen Händen oder Füßen, am Gliederstumpf werden spezielle Prothesenschäfte verwendet, die anatomisch an den Gliederstumpf angepasst sein müssen. Der Halt wird üblicherweise durch ein Vakuum zwischen Prothesenschaft und Gliederstumpf bzw. zwischen Gliederstumpf und einem Silikonliner bewirkt.

Prothesenschäfte gibt es aus Metall, Holz oder aus Gießharz. Es gibt sie mit längs- oder querovaler Schaftform, als Vollkontaktschaft, mit Ventil oder mit Silikonliner. Bei Amputationen unterhalb des Kniegelenks haben die Prothesenschäfte etwas Dreiecksform mit Halteeinrichtungen an der Knochenstruktur.

Da die Prothesenschäfte möglichst exakt an den Gliederstumpf angepasst sein müssen, um unter anderem das den Halt bewirkende Vakuum erzeugen und halten zu können, handelt es sich stets um individuelle Einzelanfertigungen, die auch eine perfekte Druckverteilung der Last auf die gesamte Stumpffläche bewirken müssen, damit der Stumpf das Körpergewicht und andere Kräfte optimal übertragen kann. Da nun nach einem Unfall oder einer Amputation der Gliederstumpf innerhalb weniger Tage oder Wochen zunächst anschwillt und dann wieder abschwillt, aus medizinischer und orthopädischer Sicht jedoch der Patient schnellstmöglich mit einer Prothese versorgt werden muss, müssen allein während der Genesungs- und Eingewöhnungszeit unter Umständen mehrere Prothesenschäfte individuell angefertigt werden. Dies ist wegen des damit verbundenen Zeit- und insbesondere Kostenaufwandes unbefriedigend.

Selbstverständlich hat es nicht an Versuchen gefehlt, diesem Missstand abzuhelfen. So wurden beispielsweise zwischen Prothesenschaft und Gliederstumpf aufblasbare Luftkammern angeordnet. Man vergleiche WO 00/23016. Es hat sich jedoch sehr schnell herausgestellt, dass der Halt der Prothese am Gliederstumpf dadurch deutlich reduziert ist. Vor allem aber war der Gebrauchswert der Prothese stark reduziert, da sie nicht mehr steif mit dem Gliederstumpf verbunden ist.

Zur Umgehung dieses Problems schlägt die DE 27 18 395 C vor, in die Luftkammern kleine Kugeln einzufüllen. Die mit den Kugeln gefüllten Luftkammern sollen sich an die Kontur des Gliederstumpfes anpassen. Durch Evakuieren der Luftkammern sollen die Kugeln anschließend in ihrer aktuellen, mehr oder weniger gut an den Gliederstumpf angepassten Position fixiert werden. Trotz dieses guten Gedankens hat sich diese Konstruktion jedoch nicht bewährt und daher in der Praxis nicht durchsetzen können.

Bereits aus der 1919 veröffentlichten DE-Patentschrift 314 985 ist ein Prothesenschaft bekannt, der aus einer Innen- und einer Außenschale zusammengesetzt ist. Innen- und Außenschale bestehen aus einzelnen Lamellen, die mittels jeweils einer Niet so untereinander verbunden sind, dass Innenschale und Außenschale im Durchmesser verändert werden können. Zur Veränderung der Durchmesser von Innenschale und Außenschale sind mehrere Spannbänder vorgesehen. Allerdings sind nach dem Schließen des der Außenschale zugeordneten Spannbandes die der Innenschale zugeordneten Spannbänder bzw. deren Verschlüsse nicht mehr zugänglich. Das Anlegen und Abnehmen eines solchen Prothesenschaftes ist daher äußerst umständlich und zeitaufwändig. Auch können aus Lamellen zusammengesetzte Prothesenschäfte kein Vakuum zum Halten des Gliederstumpfes entwickeln.

Aus der 1920 veröffentlichten DE-Patentschrift 323 671 ist ein weiterer, zweischaliger Prothesenschaft bekannt. Auch hier sind Innenschale und Außenschale wieder aus Lamellen zusammengesetzt. Dabei sind die Lamellen jeder Schale mittels einer Niet so miteinander verbunden, dass mit Hilfe von Spannbändern eine Durchmesserveränderung möglich ist. Außerdem sind die Lamellen der Innenschale an einer oberen Stelle mit den Lamellen der Außenschale beweglich verbunden. Ziel dieser Konstruktion war es, Lücken zwischen den einzelnen Lamellen zu vermeiden. Auch diese Konstruktion konnte sich in der Praxis nicht durchsetzen.

Aus der orthopädischen Praxis ist bekannt, dass Gliederstümpfe sich nicht nur in der Weite verändern, sondern auch in der Länge. Dies ist beispielsweise dann der Fall, wenn die Spitze des Gliederstumpfes, die direkten Kontakt mit der Prothese hat und beispielsweise bei einer Oberschenkelprothese das Gesamtgewicht des Patienten tragen muss, sich entzündet und anschwillt. Eine Längenänderung kann auch auftreten, wenn das nach der Amputation oder einer Operation angeschwollene Gewebe abheilt und dabei abschwillt. Der Gliederstumpf sitzt dann nicht mehr korrekt auf der in jedem Prothesenschaft vorgesehenen konischen Kontaktfläche auf und kann daher beispielsweise das Körpergewicht nicht mehr optimal auf die Prothese übertragen. Auch in diesen Fällen müssen neue. Prothesenschäfte angefertigt oder die alten Prothesenschäfte umgearbeitet werden. Das ist unbefriedigend.

Aus der DE 82 16 840 U ist ein Prothesenschaft bekannt, in dem die konische Auflagefläche für den Gliederstumpf in der Höhe verstellbar ist. Die Höhenverstellung erfolgt mit Hilfe von Hebel- oder Keilmechaniken, die der Patient von der Außenseite des Prothesenschaftes aus mit Hilfe einer Kurbel oder dergleichen betätigen kann. Die Höhenverstellung mittels eines eingebauten und gegebenenfalls mit batteriegespeisten Elektromotors ist ebenfalls vorgesehen. Da diese Höhenverstellung in den individuell angefertigten Prothesenschaft eingebaut werden muss, muss dieser entsprechend vergrößert und bearbeitet werden. Das, erhöht Zeitaufwand und Kosten.

Aus der US 6106559, ist ein Prothesenschaft bekannt mit einen Silikonliner mit Kupplungsstift, einem Prothesenschaft, einem Halter zum Verbinden der Gliedmaßen mit dem Prothesenschaft, einem Bund in dem ein Adapter befestigt ist, einer Kupplung zur lösbaren Fixierung des Kupplungsstiftes und einer Mechanik zum lösen der Kupplung.

Wie eingangs erwähnt, muss jeder Prothesenschaft so konstruiert sein, dass er die Kräfte optimal auf den Gliederstumpf überträgt. Dazu muss der Prothesenschaft entsprechend steif sein. Um den Durchmesser verändern zu können, muss der Prothesenschaft nachgiebig sein. Die Nachgiebigkeit in Umfangsrichtung kann durch den Aufbau aus Lamellen wie in den oben beschriebenen deutschen Patentschriften erreicht werden. Die Verbindung zwischen den Lamellen mit nur jeweils einer Niet hat jedoch den Nachteil, dass sie die Stabilität des Prothesenschaftes schwächt.

### Darstellung der Erfindung

Der vorliegenden Erfindung liegt daher die Aufgabe zugrunde, eine Prothese der eingangs genannten Art anzugeben, die einfach und dank Vorfertigung in größeren Serien preiswert herzustellen ist, eine hohe Festigkeit besitzt und innerhalb vorgegebener Grenzen auf Gliederstümpfe passt, deren Durchmesser und deren Länge sich verändert.

Diese Aufgabe wird gelöst durch eine Prothese mit den Merkmalen des Anspruchs 1, im wesentlichen umfassend
- einen Silikonliner mit Kupplungsstift,
- einen Prothesenschaft,
   - passend zu einem Gliederstumpf,
   - bereichsweise mit Längsschlitzen versehen
   - und mittels Spannelementen im Durchmesser veränderbar,
- und einen Halter zum Verbinden einer künstlichen Gliedmaße mit dem Prothesenschaft,
- die Längsschlitze sind überbrückt,
- der Prothesenschaft besitzt einen konzentrischen Bund,
- im Bund ist ein zylindrischer Adapter höhenverstellbar befestigt,
- der Adapter
   - trägt
      - oben eine Auflagefläche für den Gliederstumpf bzw. die Spitze des Silikonliners
      - und unten den Halter
   - und besitzt in seinem Inneren
   - oben eine Kupplung zur lösbaren Fixierung des Kupplungsstiftes
   - und unten eine Mechanik zum Lösen der Kupplung.

Gemäß der vorliegenden Erfindung wird die Nachgiebigkeit des Prothesenschaftes durch die Längsschlitze, die Steifigkeit des geschlitzten Prothesenschaftes durch die Überbrückung der Längsschlitze erreicht. Dank der erheblich verbesserten Steifigkeit kann die Wandstärke verringert und so der Tragekomfort verbessert werden. Die Einstellbarkeit des Durchmessers des Prothesenschaftes erlaubt es, mit wenigen industriell vorgefertigten Grundformen für jeweils die rechte und linke Körperseite den größten Teil aller vorkommenden Erstversorgungen ausführen zu können. Auch ist die Prothese schnell und bequem angelegt und abgenommen. Ihr Sitz ist einwandfrei.

Zum Anlegen einer Prothese wird zunächst der Silikonliner auf den Gliederstumpf aufgerollt. Anschließend müssen Gliederstumpf und Silikonliner in den Prothesenschaft der Prothese eingeführt werden, bis die Spitze des Gliederstumpfes bzw. des Silikonliners auf der konischen Auflagefläche aufsitzt, wobei der Kupplungsstift ganz in die Kupplung eintaucht. Wenn nun der Prothesenschaft sehr stramm sitzt, beispielsweise weil der Gliederstumpf etwas angeschwollen ist, kann es bei einem einteiligen Prothesenschaft passieren, dass die optimale Position nicht erreicht wird. Aus diesem Grunde haben derzeit handelsübliche Prothesen ein eingebautes Zahnrad mit Freilauf, weiches der Prothesenträger mit einer Münze von außen drehen kann. Dieses Zahnrad kämmt mit dem profilierten Kupplungsstift, der auf diese Weise in den Prothesenschaft hineingezogen wird. Bei der erfindungsgemäßen Prothese dagegen ist eine solche Konstruktion völlig entbehrlich, weil jeder Gliederstumpf ohne Schwierigkeiten in den geöffneten Prothesenschaft eingeführt werden kann. Dasselbe gilt für das Ablegen der Prothese.

Sobald ein Patient die erfindungsgemäße Interimsprothese nicht mehr benötigt, kann sie gereinigt und beim nächsten Patienten wieder verwendet werden. Die damit verbundene Reduzierung an Ressourcen und Kosten ist bis dato unerreicht.

Die Möglichkeit, die erfindungsgemäße Interimsprothese industriell in wenigen Größen vorzufertigen, ist im übrigen nur möglich, weil die erfindungsgemäße Prothese nicht nur eine Weitenverstettung sondern auch eine Längenverstellung besitzt in Form eines zylindrischen Adapters, der je nach Bedarf mehr oder weniger tief in den Prothesenschaft hineingeschoben wird. Dieser Adapter trägt an seiner Oberseite die konische Auflagefläche für den Gliederstumpf, an seiner Unterseite die Standardkupplung zur Befestigung einer künstlichen Gliedmaße und in seinem Inneren die Mechanik zum lösbaren Halten des Kupplungsstiftes der handelsüblichen Silikonliner.

Gemäß einer ersten Ausgestaltung der Erfindung sind die Längsschlitze wenigstens teilweise mit einem nachgiebigen Material überbrückt, das sich in Wellen oder Falten legt. Diese Wellen und Falten können aus demselben Material gebildet werden, aus dem auch der Prothesenschaft besteht, wobei die Nachgiebigkeit durch geschickte Auswahl von Wandstärke und Form erreicht wird.

Alternativ hierzu besteht auch die Möglichkeit, die Längsschlitze durch ein gummiartiges Material, insbesondere ein Silikongummi zu überbrücken.

Eine bevorzugte, besonders stabile Ausgestaltung der Erfindung sieht vor, dass der Prothesenschaft durch zwei konzentrische Schalen gebildet ist. Dabei entspricht die Innenfläche der Außenschale im wesentlichen der Außenfläche der Innenschale, die Längsschlitze von Außen- und Innenschale sind gegeneinander versetzt und die Durchmesser beider Schalen sind durch ein Spannelement gemeinsam veränderbar. Außerdem besitzen Außen- und Innenschale einen konzentrischen Bund, der den Adapter hält.

Diese Ausgestaltung der Erfindung besitzt auch bei minimalen Wandstärken der Schalen, die passgenau zueinander hergestellt sind, vergleichbar den Schalen einer Zwiebel, eine hohe Steifigkeit, verbunden mit geringem Gewicht und hohem Tragekomfort. Da beide Schalen nur von einer Art Spannelement gehalten werden, ist auch eine solche Prothese sehr schnell angelegt und abgenommen. Auch lässt sich diese Ausgestaltung der Erfindung in wenigen Grundformen industriell vorfertigen, wodurch sich die Kosten der Prothese zusätzlich reduzieren.

Gemäß einer vorteilhaften Ausgestaltung der Erfindung ist der Kupplungsstift am Silikonliner ein glatter Zylinder und die Kupplung besteht aus einer Klemmplatte mit einer Öffnung, passend zum Kupplungsstift, wobei die Klemmplatte an einer Achse schwenkbar gelagert ist und durch Federn in die Klemmstellung geschwenkt wird. Ein solcher glatter Kupplungsstift ist nicht nur einfacher und preiswerter als ein profilierter Kupplungsstift, sondern lässt sich auch in allen Fällen leicht lösen, ganz im Gegensatz zu den herkömmlichen profilierten Stiften, die von einem Zahnrad mit Freilauf gehalten werden, das nach längerem Tragen der Prothese unter einer enormen Vorspannung steht und daher gerne klemmt.

Vorteilhafterweise ist die wegen der Kürze des Kupplungsstiftes im oberen Teil des Adapters positionierte Klemmplatte mittels eines Seils oder einer Speiche mit einem Handhebel verbunden, der am unteren Ende des Adapters positioniert und deshalb bei jeder Höhenstellung gut zugänglich ist. Grundsätzlich arbeitet diese Kupplungskonstruktion auch mit den handelsüblichen profilierten Kupplungsstiften zusammen, jedoch ist dann die Höhenverstellung nicht stufenlos.

Vorteilhafterweise besteht der Adapter aus zwei Teilen, die durch Schrauben oder dergleichen zusammengehalten werden. Dabei kann gemäß einer vorteilhaften Ausgestaltung eine der Befestigungsschrauben gleichzeitig als Schwenkachse für die Klemmplatte der Kupplung eingesetzt werden.

Die Verbindung zwischen Innen- und Außenschale und Adapter erfolgt vorzugsweise durch eine Klemmverbindung. Um eine sichere Klemmverbindung zwischen Prothesenschaft und Adapter zu erzielen, empfiehlt es sich, den Bund federnd zu schlitzen.

Der Klemmring selbst besitzt gemäß einer bevorzugten Ausgestaltung der Erfindung Flansche zum Ansetzen von Spannschrauben.

Wie erwähnt ermöglicht der erfindungsgemäße Adapter eine Anpassung an unterschiedlich lange Gliederstümpfe oder auch solche, die aus medizinischen Gründen verkürzt werden mussten, ohne dass der eigentliche Prothesenschaft verändert werden muss. Verlängert werden muss lediglich das Verbindungsrohr zwischen der künstlichen Gliedmaße und dem am unteren Ende des Adapters angebrachten handelsüblichen Halter.

Gemäß einer Ausgestaltung der Erfindung umfasst die Mechanik zum Lösen der Kupplung einen Handhebel, der einklappbar gestaltet ist, so dass er beim Tragen der Prothese nicht stört.

Vorteilhafterweise bestehen die Schalen des Prothesenschaftes aus faser-, insbesondere kohlefaserverstärktem Kunststoff. Dabei erstrecken sich die Verstärkungsfasern im wesentlichen in Achsrichtung, so dass wie gewünscht eine hohe Steifigkeit in Achsrichtung und eine ausreichende Nachgiebigkeit in Umfangsrichtung besteht.

Für den Fall, dass die Längsschlitze in der Außen- oder Innenschale den Patienten stören, können diese wenigstens teilweise mit einem nachgiebigen Material gefüllt werden. Dieses nachgiebige Material kann ein gummiartiger Stoff sein, beispielsweise ein Silikongummi.

Um die erfindungsgemäße Prothese schnell anlegen und abnehmen zu können, besteht das Spannelement bevorzugt aus einem Spannband mit Kniehebelverschluss.

Um nun zu verhindern, dass es zu einer Blutstauung im Gliederstumpf kommt, weil das Spannband zu eng gestellt ist, oder dass die Prothese nicht richtig sitzt, weil das Spannband zu weit gestellt ist, ist das Spannelement mit einer Längenverstellung ausgerüstet, die nur vom Fachmann, beispielsweise vom Orthopädiemechaniker, zu betätigen ist. Der Patient selbst kann dann das Spannelement lediglich öffnen und schließen.

Schließlich sollte sichergestellt sein, dass Innenschale und Außenschale, Adapter und gegebenenfalls Klemmring gegen Verdrehen gesichert sind.

### Kurze Beschreibung der Zeichnung

Anhand der Zeichnung soll die Erfindung in Form eines Ausführungsbeispiels näher erläutert werden. Es zeigen jeweils rein schematisch
- Fig. 1: einen in Umfangsrichtung und in Längsrichtung verstellbaren Prothesenschaft,
- Fig. 2: in vergrößertem Maßstab ein Stück des Prothesenschaftes der Fig. 1 mit eingesetztem Adapter in ganz ausgefahrener Position,
- Fig. 3: den Adapter der Fig. 2 in seiner ganz eingefahrenen Position,
- Fig. 4: den Adapter der Fig. 2 und 3 in geöffnetem Zustand und
- Fig. 5: verschiedene Querschnitte von Falten oder Wellen.

### Wege zur Ausführung der Erfindung

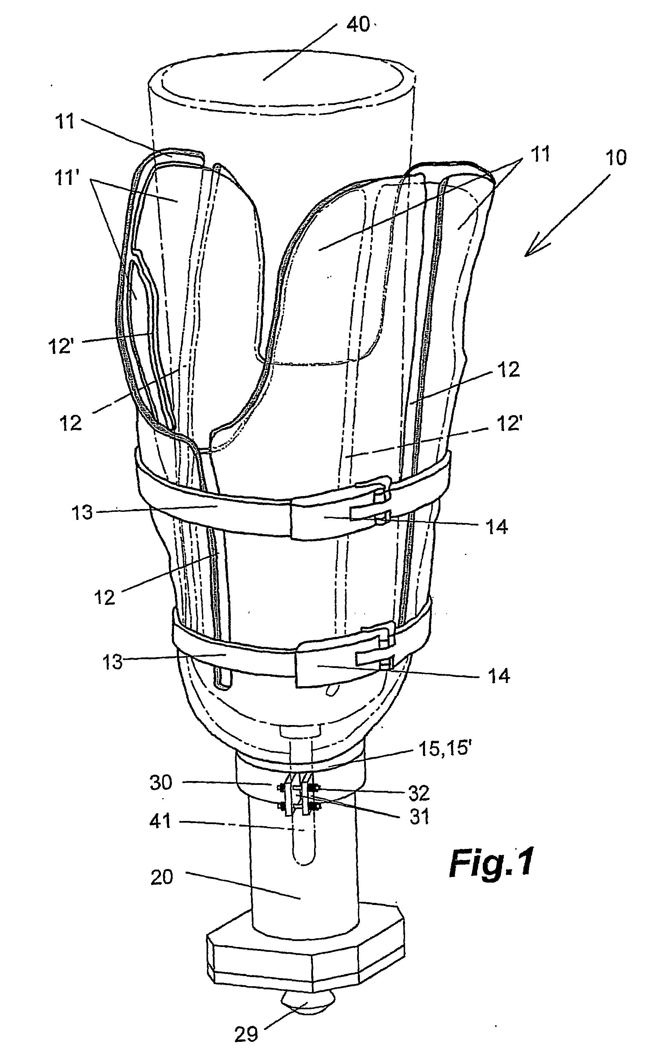

Fig. 1 zeigt in perspektivischer Darstellung einen an einen Gliederstumpf (nicht dargestellt) anatomisch angepassten Prothesenschaft 10, an dem unten eine Kupplung 29 angebracht ist, an der gegebenenfalls über ein Verlängerungsrohr eine künstliche Gliedmaße, z. B. eine Arm- oder Fußprothese, angebracht werden kann.

Der Prothesenschaft 10 besteht aus einer Außenschale 11 und einer Innenschale 11'. In Außen- und Innenschale 11,11' sind im wesentlichen in Achsrichtung verlaufende Schlitze 12, 12' eingearbeitet, wobei diese gegeneinander versetzt sind. Im Idealfall berühren sich Außen- und Innenschale 11,11' wie die Schalen einer Zwiebel.

Werden Außen- und Innenschale 11, 11' aus kohlefaserverstärktem Kunststoff hergestellt, so lassen sich dünne Wandungen und damit geringe Gewichte erreichen. Verlaufen die Verstärkungsfasern überwiegend in Achsrichtung, sind die Schalen 11, 11' in Längsrichtung steif, in Umfangsrichtung nachgiebig.

Um den Durchmesser des aus Außen- und Innenschale 11, 11' bestehenden Prothesenschaftes 10 verändern zu können, sind zwei Spannbänder 13 mit Kniehebelverschluss 14 vorgesehen, deren Weite der Fachmann mittels Feineinstellung (nicht dargestellt) verändern kann. Beim Schließen der Kniehebelverschlüsse 14 werden die Schlitze 12, 12' zusammengefahren, worauf der Prothesenschaft 10 sich eng an den Gliederstumpf anlegt.

In Fig. 1 ist rein schematisch auch ein Silikonliner 40 eingezeichnet. Dieser wird zunächst auf den Gliederstumpf aufgezogen, auf dem er durch Vakuum fest sitzt. An der Unterseite des Silikonliners 40 ist ein Kupplungsstift 41 vorgesehen, der mit einer Kupplung zusammenwirkt. Diese Kupplung sitzt im Inneren eines zylinderförmigen Adapters 20, der mit Hilfe eines Klemmrings 30 höhenverstellbar im Prothesenschaft 10 fixiert ist. Zu diesem Zweck besitzen Außen- und Innenschale 11, 11' an ihrem unteren Ende je einen konzentrischen Bund 15, 15'. Dank eines Schlitzes 16 ist der Bund 15, 15' federnd nachgiebig, Der Klemmring 30 besitzt Flansche 31 und kann mit Hilfe von Spannschrauben 32 gespannt bzw. gelöst werden.

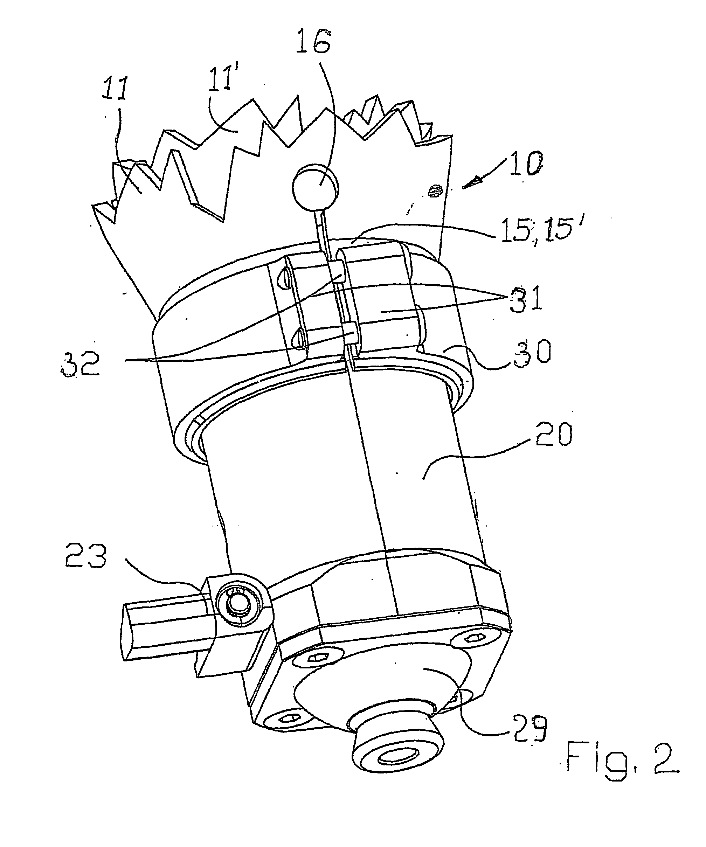

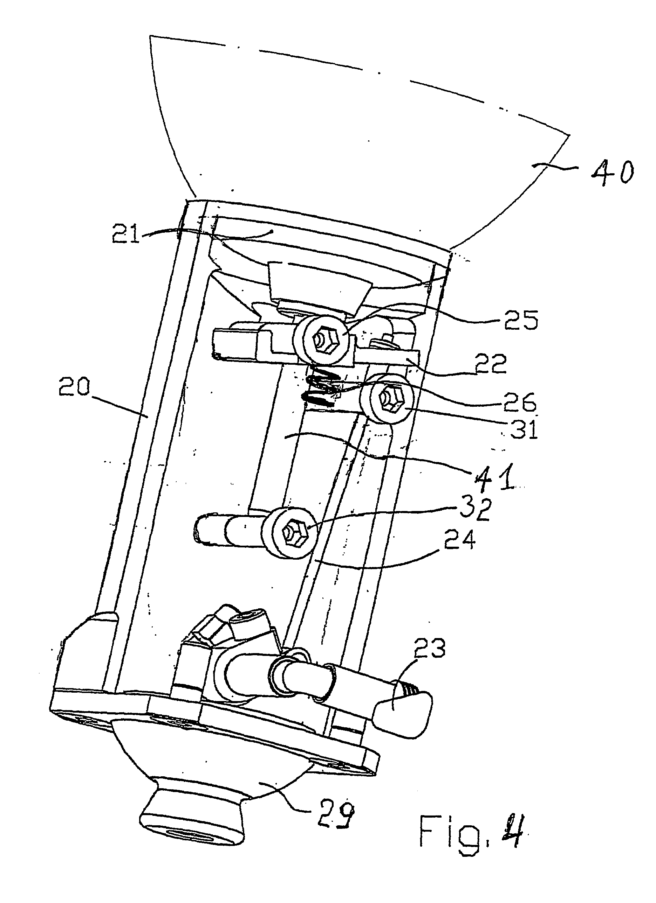

Fig. 2 zeigt in vergrößertem Maßstab einen Ausschnitt aus Fig. 1. Man erkennt den Adapter 20 in seinem ganz ausgefahrenen Zustand. Des weiteren erkennt man am unteren Ende des Adapters 20 einen ein- und ausklappbaren Handhebel 23, mit dessen Hilfe die im Inneren des Adapters 20 untergebrachte Kupplung betätigt wird. Einzelheiten sollen anhand der Fig. 4 erläutert werden.

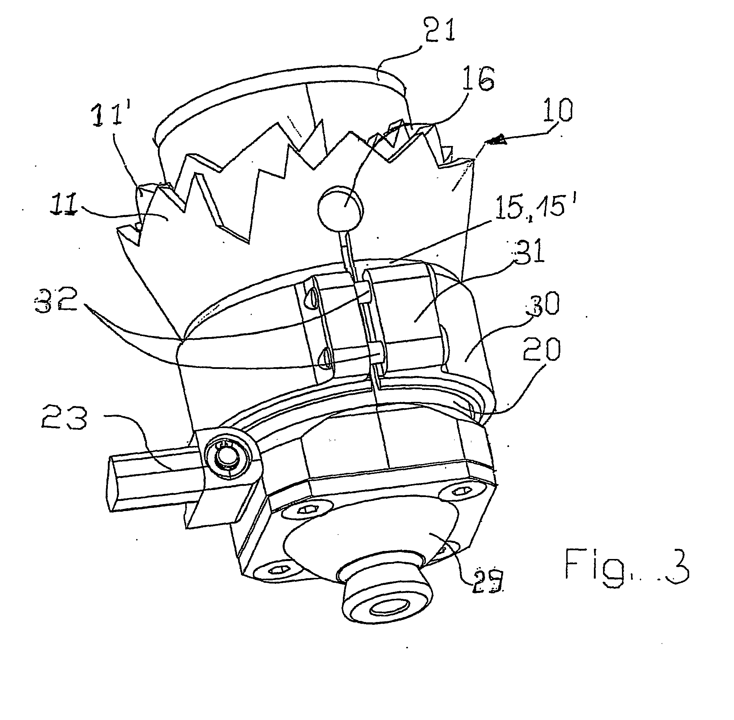

Fig. 3 zeigt den Adapter der Fig. 2 in seinem ganz in den Prothesenschaft 10 eingefahrenen Zustand. Dadurch wird am oberen Ende des Adapters 20 eine konische Auflagefläche 21 für den Gliederstumpf bzw. den Silikonliner 40 erkennbar.

Fig. 4 zeigt den Adapter 20 in geöffnetem Zustand. Man erkennt an seiner Oberseite die konische Auflagefläche 21, auf der die Spitze des Silikonliners 40 aufsitzt. Dessen Kupplungsstift 41 ist als glatter Zylinder ausgebildet und taucht durch die Auflagefläche 21 hindurch. Die Kupplung wird gebildet durch eine Klemmplatte 22, die um eine Achse 25, die gleichzeitig als Verbindungsschraube ausgebildet ist, schwenkbar gelagert ist. Federn 26 schwenken die Klemmplatte 22 in eine Klemmposition derart, dass der Kupplungsstift 41 problemlos eingefahren werden kann und gleichzeitig gegen ein ungewolltes Lösen gesichert ist. Weitere Schrauben 32 verbinden die Teile des Adapters 20.

Zum Lösen der Klemmplatte 22 dient der Handhebel 23, der über eine im Inneren des Adapters 20 untergebrachte Koppelstange 24 bzw. ein Koppelseil mit der Klemmplatte 22 in Verbindung steht. Schwenkt der Patient den Handhebel 23 nach unten, so wird auch die Klemmplatte 22 aus ihrer in der Zeichnung dargestellten Klemmlage nach unten in die Neutralstellung geschwenkt und der Patient kann den Kupplungsstift 41 mit Silikonliner 40 und Gliederstumpf vom Adapter 20 bzw. Prothesenschaft 10 abnehmen, sobald die Spannbänder 13, geöffnet sind. Bei der Klemmplatte 22 handelt es sich um eine einfache, gehärtete Stahlplatte mit einer Bohrung, die auf den Durchmesser des Kupplungsstiftes 41 abgestimmt ist. Diese einfache Konstruktion ist äußerst dauerhaft und betriebssicher.

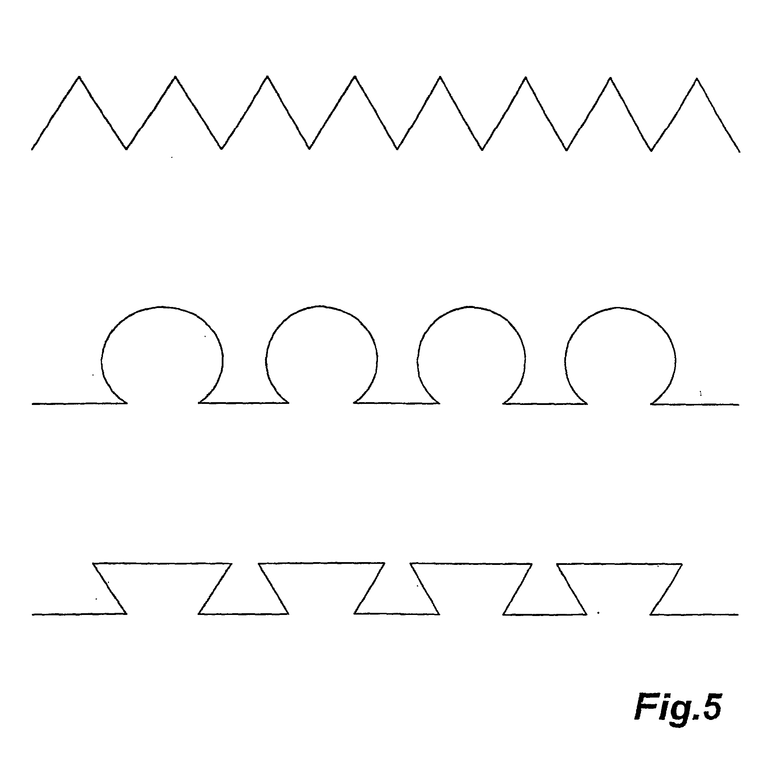

Fig. 5 zeigt rein schematisch drei mögliche Querschnitte von Falten bzw. Wellen, mit denen sich die Längsschlitze 12 überbrücken lassen. Diese Querschnitte sind von oben nach unten zickzack-förmig, omega-förmig und schwalbenschwanzförmig.

## Patentansprüche

1. Prothese, im wesentlichen umfassend
- einen Silikonliner (40) mit Kupplungsstift (41),
- einen Prothesenschaft (10),
- passend zu einem Gliederstumpf,
- bereichsweise mit Längsschlitzen (12) versehen
- und einen Halter (29) zum Verbinden einer künstlichen Gliedmaße mit dem Prothesenschaft (10),
- der Prothesenschaft (10) besitzt einen konzentrischen Bund (15),
- im Bund (15) ist ein zylindrischer Adapter (20) höhenverstellbar befestigt,
- der Adapter (20)
- trägt
- oben eine Auflagefläche (21) für den Gliederstumpf bzw. die Spitze des Silikonliners (40)
- und unten den Halter (29)
- und besitzt in seinem Inneren
- oben eine Kupplung (22-26) zur lösbaren Fixierung des Kupplungsstiftes (41)
- und unten eine Mechanik (23) zum Lösen der Kupplung (22-26),
**gekennzeichnet durch** die Merkmale:
- die Längsschlitze (12) sind überbrückt,
- und der Prothesenschaft ist mittels Spannelementen (13, 14) im Durchmesser veränderbar,

2. Prothese nach Anspruch 1, **gekennzeichnet durch** das Merkmal:
- die Längsschlitze (12) sind wenigstens teilweise mit einem nachgiebigen Material überbrückt, das sich in Wellen oder Falten legt.

3. Prothese nach Anspruch 1 oder 2, **gekennzeichnet durch** die Merkmale:
- der Prothesenschaft (10) ist gebildet **durch** zwei konzentrische Schalen (11, 11'),
- die Innenfläche der Außenschale (11) entspricht im wesentlichen der Außenfläche der Innenschale (11'),
- die Längsschlitze (12, 12') von Außen- und Innenschale (11, 11') sind gegeneinander versetzt,
- die Durchmesser beider Schalen (11, 11') sind **durch** ein Spannelement (13, 14) gemeinsam veränderbar, Außen- und Innenschale (11, 11') besitzen einen konzentrischen Bund (15, 15').

4. Prothese nach einem der Ansprüche 1 bis 3, **gekennzeichnet durch** die Merkmale:
- der Kupplungsstift (41) ist ein glatter Zylinder,
- die Kupplung umfasst
- eine Klemmplatte (22) mit einer Öffnung, passend zum Kupplungsstift (41),
- eine Achse (25), an der die Klemmplatte (22) schwenkbar gelagert ist,
- und Federn (26), die die Klemmplatte (22) in ihre Klemmstellung schwenken.

5. Prothese nach einem der Ansprüche 1 bis 4, **gekennzeichnet durch** die Merkmale:
- der Adapter (20) besteht aus zwei Teilen,
- die Achse (25) dient gleichzeitig als Verbindungselement.

6. Prothese nach einem der Ansprüche 1 bis 5, **gekennzeichnet durch** das Merkmal:
- der Bund (15, 15') der Schalen (11, 11') ist federnd geschlitzt.

7. Prothese nach einem der Ansprüche 1 bis 6, **gekennzeichnet durch** das Merkmal:
- auf den Bund (15, 15') ist ein Klemmring (30) aufgesetzt.

8. Prothese nach Anspruch 7, **gekennzeichnet durch** das Merkmal:
- der Klemmring (30) besitzt Flansche (31) zum Ansetzen von Klemmschrauben (32).

9. Prothese nach einem der Ansprüche 1 bis 8, **gekennzeichnet durch** das Merkmal:
- die Mechanik zum Lösen der Kupplung (22-26) umfasst einen Handhebel (23),
- der Handhebel (23) ist einklappbar.

10. Prothese nach einem der Ansprüche 1 bis 9, **gekennzeichnet durch** das Merkmal:
- die Schalen (11, 11') bestehen aus faser-, insbesondere kohlefaserverstärktem Kunststoff.

11. Prothese nach Anspruch 10, **gekennzeichnet durch** das Merkmal:
- die Verstärkungsfasern erstrecken sich im wesentlichen in Achsrichtung.

12. Prothese nach einem der Ansprüche 1 bis 11, **gekennzeichnet durch** das Merkmal:
- die Schlitze (12, 12') in der Außenschale (11) und/oder Innenschale (11') sind wenigstens teilweise mit einem gummiartigen Material, insbesondere Silikongummi überbrückt.

13. Prothese nach einem der Ansprüche 1 bis 12, **gekennzeichnet durch** das Merkmal:
- das Spannelement besteht aus einem Spannband (13) mit Kniehebelverschluss (14).

14. Prothese nach einem der Ansprüche 1 bis 13, **gekennzeichnet durch** das Merkmal:
- das Spannelement (13, 14) besitzt eine längenverstellung, die nur vom Fachmann zu betätigen ist.

15. Prothese nach einem der Ansprüche 1 bis 14, **gekennzeichnet durch** das Merkmal:
- Innenschale (11') und Außenschale (11), Adapter (20) und gegebenenfalls Klemmring (30) sind gegen Verdrehen gesichert.

## Claims

1. A prosthesis substantially comprising
- a silicone liner (40) with a coupling pin (41),
- a prosthesis shaft (10),
- suitable for a limb stump,
- provided in zones with longitudinal slits (12)
- and a holder (29) for connecting an artificial limb to the prosthesis shaft (10),
- the prosthesis shaft (10) has a concentric collar (15),
- a cylindrical adapter (20) is secured vertically adjustably in the collar (15),
- the adapter (20)
- carries
- at the top a support surface (21) for the limb stump and/or the tip of the silicone liner (40)
- and at the bottom the holder (29)
- and has in its interior
- at the top a coupling (22-26) for the releasable fixing of the coupling pin (41)
- and at the bottom a mechanism (23) for releasing the coupling (22-26),
**characterised by** the features:
- the longitudinal slits (12) are bridged
- and the prosthesis shaft (10) can be varied in diameter by means of tightening elements (13,14).

2. A prosthesis according to Claim 1, **characterised by** the feature:
- the longitudinal slits (12) are bridged at least partly by a resilient material which lies in undulations or folds.

3. A prosthesis according to Claim 1 or 2, **characterised by** the features:
- the prosthesis shaft (10) is formed by two concentric shells (11,11'),
- the inner surface of the outer shell (11) corresponds substantially to the outer surface of the inner shell (11'),
- the longitudinal slits (12,12') of the outer and inner shells (11,11') are offset relative to one another,
- the diameters of both shells (11,11') are mutually variable by means of a tightening element (13,14), the outer and inner shells (11,11') have a concentric collar (15,15').

4. A prosthesis according to any one of Claims 1 to 3, **characterised by** the features:
- the coupling pin (41) is a smooth cylinder,
- the coupling comprises
- a clamping plate (22) with an opening fitting the coupling pin (41),
- a spindle (25) on which the clamping plate (22) is pivotably mounted,
- and springs (26) which pivot the clamping plate (22) into its clamping position.

5. A prosthesis according to any one of Claims 1 to 4, **characterised by** the features:
- the adapter (20) consists of two parts,
- the spindle (25) serves simultaneously as a connecting element.

6. A prosthesis according to any one of Claims 1 to 5, **characterised by** the feature:
- the collar (15,15') of the shells (11,11') is flexibly slotted.

7. A prosthesis according to any one of Claims 1 to 6, **characterised by** the feature:
- a clamping ring (30) is mounted the collar (15,15').

8. A prosthesis according to Claim 7, **characterised by** the feature:
- the clamping ring (30) has flanges (31) for the application of clamping screws (32).

9. A prosthesis according to any one of Claims 1 to 8, **characterised by** the feature:
- the mechanism for releasing the coupling (22-26) comprises a hand lever (23),
- the hand lever (23) can be folded in.

10. A prosthesis according to any one of Claims 1 to 9, **characterised by** the feature:
- the shells (11,11') consist of fibre-reinforced, in particular carbon fibre-reinforced plastics material.

11. A prosthesis according to Claim 10, **characterised by** the feature:
- the reinforcing fibres extend substantially in an axial direction.

12. A prosthesis according to any one of Claims 1 to 11, **characterised by** the feature:
- the slits (12,12') in the outer shell (11) and/or the inner shell (11') are bridged at least partly by a rubber-like material, in particular silicone rubber.

13. A prosthesis according to any one of Claims 1 to 12, **characterised by** the feature:
- the tightening element comprises a retaining strap (13) with a toggle lever fastener (14).

14. A prosthesis according to any one of Claims 1 to 13, **characterised by** the feature:
- the tightening element (13,14) has a longitudinal adjustment means which is to be actuated only by the expert.

15. A prosthesis according to any one of Claims 1 to 14, **characterised by** the feature:
- the inner shell (11') and outer shell (11), the adapter (20) and, optionally, the clamping ring (30) are secured against rotation.

## Revendications

1. Prothèse comprenant pour l'essentiel
- un manchon en silicone (40) avec une tige d'emboîture (41),
- une tige de prothèse (10),
- adaptée à un moignon,
- munie de fentes longitudinales (12) à certains endroits
- et la tige de prothèse (10) est de diamètre réglable grâce à des éléments de serrage (13, 14)
- et un support (29) pour relier une masse de membre artificielle à la tige de prothèse (10),
**caractérisée par** les éléments suivants :
- les fentes longitudinales (12) sont maintenues par des bandes,
- la tige de prothèse (10) possède un collet concentrique (15),
- dans le collet (15) est fixé un adaptateur cylindrique (20) de hauteur réglable,
- l'adaptateur (20)
- supporte
- en haut, une surface de contact (21) pour le moignon ou la pointe du manchon en silicone (40)
- et en bas, le support (29)
- et possède à l'intérieur
- en haut, une emboîture (22-26) pour fixer de manière amovible la tige d'emboîture (41)
- et en bas, un mécanisme (23) permettant de dégager l'emboîture (22 - 26),

2. Prothèse selon la revendication 1, **caractérisée par** l'élément suivant :
- les fentes longitudinales (12) sont maintenues au moins partiellement par un matériau souple appliqué en ondulations ou en plis.

3. Prothèse selon la revendication 1 ou 2, **caractérisée par** les éléments suivants :
- la tige de prothèse (10) est formée de deux coques concentriques (11, 11'),
- la face interne de la coque extérieure (11) coïncide pour l'essentiel avec la face externe de la coque intérieure (11')
- les fentes longitudinales (12, 12') de la coque extérieure et de la coque intérieure (11, 11') sont décalées l'une par rapport à l'autre,
- le diamètre des deux coques (11, 11') peut être modifié grâce à un élément de serrage (13, 14),
- la coque extérieure et la coque intérieure (11, 11') possèdent un collet concentrique (15, 15').

4. Prothèse selon l'une des revendications 1 à 3, **caractérisée par** les éléments suivants :
- la tige d'emboîture (41) est un cylindre lisse,
- l'emboîture comprend
- une plaque de serrage (22) comportant une
- ouverture, adaptée à la tige d'emboîture (41),
- un axe (25) autour duquel la plaque de serrage (22) peut pivoter,
- et des ressorts (26) qui orientent la plaque de serrage (22) dans sa position de serrage.

5. Prothèse selon l'une des revendications 1 à 4, **caractérisée par** les éléments suivants :
- l'adaptateur (20) est en deux parties,
- l'axe (25) sert en même temps d'élément de liaison.

6. Prothèse selon l'une des revendications 1 à 5, **caractérisée par** l'élément suivant :
- le collet (15, 15') des coques (11, 11') possède une fente antivibratoire.

7. Prothèse selon l'une des revendications 1 à 6, **caractérisée par** l'élément suivant :
- le collet (15, 15') est entouré d'une bague de serrage (30).

8. Prothèse selon la revendication 7, **caractérisée par** l'élément suivant :
- la bague de serrage (30) présente des brides (31) permettant de recevoir des vis de serrage (32).

9. Prothèse selon l'une des revendications 1 à 8, **caractérisée par** les éléments suivants :
- le mécanisme permettant de dégager l'emboîture (22-26) comprend un levier à main (23),
- le levier à main (23) est rabattable.

10. Prothèse selon l'une des revendications 1 à 9, **caractérisée par** l'élément suivant :
- les coques (11, 11') sont en matériau synthétique renforcé de fibres, en particulier de fibres de carbone.

11. Prothèse selon la revendication 10, **caractérisée par** l'élément suivant :
- les fibres de renfort sont orientées pour l'essentiel selon la direction de l'axe.

12. Prothèse selon l'une des revendications 1 à 11, **caractérisée par** l'élément suivant :
- les fentes (12,12') de la coque extérieure (11) et/ou de la coque intérieure (11') sont au moins partiellement maintenues par un matériau caoutchouteux, en particulier du caoutchouc de silicone.

13. Prothèse selon l'une des revendications 1 à 12, **caractérisée par** l'élément suivant :
- l'élément de serrage se compose d'une bande de serrage (13) munie d'une fermeture à genouillère (14).

14. Prothèse selon l'une des revendications 1 à 13, **caractérisée par** l'élément suivant :
- l'élément de serrage (13, 14) dispose d'un réglage de la longueur dont l'utilisation est réservée à l'homme du métier.

15. Prothèse selon l'une des revendications 1 à 14, **caractérisée par** l'élément suivant :
- la coque intérieure (11') et la coque extérieure (11), l'adaptateur (20) et, le cas échéant, la bague de serrage (30) sont protégés contre la torsion.
